# EUROPEAN PATENT SPECIFICATION

(11) **EP 3 630 093 B1**
(45) Date of publication and mention of the grant of the patent: **20.08.2025**
(21) Application number: 18733182.2
(22) Date of filing: 30.05.2018
(51) Int. Cl.: A61K 31/36, A61K 31/366, A61K 31/436, A61K 45/06, A61P 35/00, A61P 43/00

(54) **TREATMENT OF DISEASES ASSOCIATED WITH A DYSREGULATION OF THE MTOR PATHWAY**
BEHANDLUNG VON ERKRANKUNGEN IM ZUSAMMENHANG MIT EINER DYSREGULATION DES MTOR-WEGS
TRAITEMENT DE MALADIES ASSOCIÉES À UNE DERÉGULATION DE LA VOIE MTOR

(30) Priority: 30.05.2017 EP 17305620
(43) Date of publication of application: 08.04.2020
(73) Proprietor: Biocodex, 94250 Gentilly (FR)
(72) Inventor: RIBAN, Véronique, 60200 Compiegne (FR); VERLEYE, Marc, 60190 Remy (FR); LE GUERN, Marie-Emmanuelle, 60200 Compiegne (FR)
(74) Representative: Vial, Lionel
(86) International application number: PCT/EP2018/064282
(87) International publication number: WO 2018/220068

(56) References cited:
- WO-A1-2014/115764
- GB-A- 2 531 281
- US-A1- 2010 144 863
- US-A1- 2016 166 514
- TEGUH HARYO SASONGKO ET AL: "Rapamycin and its analogues (rapalogs) for Tuberous Sclerosis Complex-associated tumors: a systematic review on non-randomized studies using meta-analysis", ORPHANET JOURNAL OF RARE DISEASES, BIOMED CENTRAL LTD, LO, vol. 10, no. 1, 12 August 2015 (2015-08-12), pages 95, XP021228429, ISSN: 1750-1172, DOI: 10.1186/S13023-015-0317-7
- DUDLEY W. LAMMING ET AL: "Rapalogs and mTOR inhibitors as anti-aging therapeutics", JOURNAL OF CLINICAL INVESTIGATION, vol. 123, no. 3, 1 March 2013 (2013-03-01), pages 980 - 989, XP055209735, ISSN: 0021-9738, DOI: 10.1172/JCI64099
- KRYMSKAYA VERA P. ET AL: "PI3K/mTORC1 activation in hamartoma syndromes: Therapeutic prospects", CELL CYCLE, vol. 8, no. 3, 22 July 2013 (2013-07-22), US, pages 403 - 413, XP093076868, ISSN: 1538-4101, Retrieved from the Internet <URL:https://www.ncbi.nlm.nih.gov/pmc/articles/PMC3718392/pdf/nihms481471.pdf> DOI: 10.4161/cc.8.3.7555
- THE STICLO STUDY GROUP CHIRON C ET AL: "Stiripentol in severe myoclonic epilepsy in infancy: a randomised placebo-controlled syndrome-dedicated trial", THE LA, THE LANCET PUBLISHING GROUP, GB, vol. 356, no. 9242, 11 November 2000 (2000-11-11), pages 1638 - 1642, XP004737860, ISSN: 0140-6736, DOI: 10.1016/S0140-6736(00)03157-3
- A. YOUNES ET AL: "Utility of mTOR Inhibition in Hematologic Malignancies", THE ONCOLOGIST, vol. 16, no. 6, 31 May 2011 (2011-05-31), US, pages 730 - 741, XP055402921, ISSN: 1083-7159, DOI: 10.1634/theoncologist.2010-0318
- A. SHRIVASTAVA ET AL: "Cannabidiol Induces Programmed Cell Death in Breast Cancer Cells by Coordinating the Cross-talk between Apoptosis and Autophagy", MOLECULAR CANCER THERAPEUTICS, vol. 10, no. 7, 12 May 2011 (2011-05-12), US, pages 1161 - 1172, XP055418898, ISSN: 1535-7163, DOI: 10.1158/1535-7163.MCT-10-1100
- SANDRA L. DABORA ET AL: "Multicenter Phase 2 Trial of Sirolimus for Tuberous Sclerosis: Kidney Angiomyolipomas and Other Tumors Regress and VEGF- D Levels Decrease", PLOS ONE, vol. 6, no. 9, 6 September 2011 (2011-09-06), pages e23379, XP055418900, DOI: 10.1371/journal.pone.0023379
- MATT KAEBERLEIN: "mTOR Inhibition: From Aging to Autism and Beyond", SCIENTIFICA, vol. 2013, 1 January 2013 (2013-01-01), pages 1 - 17, XP055418968, DOI: 10.1155/2013/849186

## Description

### Field of the invention

The present invention relates to compositions useful for preventing and treating disease associated with a dysregulation of the mTOR pathway which is a hamartoma syndrome.

### Technical background

Tuberous sclerosis is an autosomal dominant genetic disorder which affects multiple organs with the formation of benign tumors principally in the brain, heart, kidney, intestine, skin and lungs. The incidence of this disorder is approximately 1 per 6000 individuals. Tuberous sclerosis mainly occurs through mutations in the tumor suppressor genes TSC1, which codes for hamartin, or TSC2, which codes for tuberin.

The tumor suppressor genes TSC1 and TSC2 act as negative regulators of the mammalian Target Of Rapamycin (mTOR) pathway which plays an essential role in cellular regulation processes, including cell growth, proliferation and survival, as well as protein translation. As a consequence, the loss of function of either TSC1 or TSC2 may lead to a hyperactivity of the mTOR pathway, which itself leads to improperly regulated cell growth, abnormal differentiation, cell proliferation and tumorigenesis.

The tumors created by the dysregulation of the mTOR pathway are generally hamartomas which do not metastasize. However, morbidity and mortality associated with hamartomas may be significant depending on their location.

Current therapeutic agents prescribed to individuals with tuberous sclerosis principally aim at alleviating or suppressing the symptoms, but do not affect the course of these disorders. These agents notably include rapamycin, everolimus (Serra et. Al. (2013) Forum Med. Suisse, 13: 696-702) and sirolimus (Dabora et al. (2011) Plos One, 6 : 1-16).

Thus, there is still a need for alternative therapeutic agents to address mTOR dysregulation and in particular tuberous sclerosis or its cause.

Stiripentol (Diacomit, 1-penten-3-ol,1-(1,3-benzodioxol)-4,4-dimethyl or 4-dimethyl-1-[3,4-methylenedioxy-3,4)-phenyl]-1-penten-3-ol) is a racemic allylic alcohol that is structurally unrelated to other antiepileptic drugs.

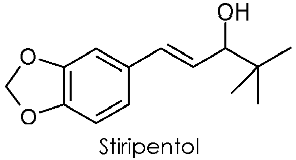

Stiripentol has shown anticonvulsant activity in several animal models but its spectrum of clinical activity is relatively narrow. Stiripentol has exhibited a high response rate in SMEI patients at a dose of 50 mg/kg/day. Recently, stiripentol has shown high efficacy in two double blind controlled clinical trials and has received approval from the European Medicines Agency (Chiron (2000) Lancet 356:1638, 2000).

### Summary of the invention

The present invention arises from the unexpected finding that stiripentol can inhibit pharmacologically activated mTOR pathway in adult rats.

Thus, the present invention is as defined in claims 1 to 13.

The present invention thus relates to a compound of the following formula (I): 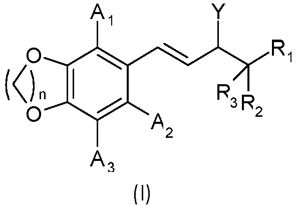 wherein:
- n represents 1 or 2,
- A₁, A₂ and A₃, which may be identical or different, represent a hydrogen atom, a halogen atom or a linear or branched alkyl group having from 1 to 4 carbon atoms,
- R₁, R₂ and R₃ represent independently a hydrogen atom or a linear or branched alkyl group having from 1 to 4 carbon atoms, and
- Y represents -OH, =O or -SH;

or a pharmaceutically acceptable salt, hydrate or solvate thereof,
for use in the prevention or treatment of a disease associated with a dysregulation of the mTOR pathway in an individual, wherein the disease associated with a dysregulation of the mTOR pathway is a hamartoma syndrome.

The present description also relates to a composition for use in a method for the prevention or treatment of a disease associated with a dysregulation of the mTOR pathway in an individual, comprising administering to the individual a prophylactically or therapeutically effective quantity of at least one compound of formula (I) as defined above or a pharmaceutically acceptable salt, hydrate or solvate thereof, wherein the disease associated with a dysregulation of the mTOR pathway is a hamartoma syndrome.

In a non-claimed aspect, the present description also relates to the use of a compound of formula (I) as defined above or a pharmaceutically acceptable salt, hydrate or solvate thereof, for the manufacture of a medicament intended for the prevention or treatment of a disease associated with a dysregulation of the mTOR pathway, wherein the disease associated with a dysregulation of the mTOR pathway is a hamartoma syndrome.

The present invention also relates to the compound of formula (I) as defined above or a pharmaceutically acceptable salt, hydrate or solvate thereof for use as defined above, in combination with at least one other inhibitor of the mTOR pathway.

The present invention also relates to a pharmaceutical composition, comprising as active substance at least one compound of formula (I) as defined above, or a pharmaceutically acceptable salt, hydrate or solvate thereof, and optionally at least one pharmaceutically acceptable carrier or excipient, for use in the prevention or treatment of a disease associated with a dysregulation of the mTOR pathway in an individual, wherein the disease associated with a dysregulation of the mTOR pathway is a hamartoma syndrome.

In an embodiment of the invention, the pharmaceutical composition for use as defined above, further comprises at least one other inhibitor of the mTOR pathway, as an active ingredient.

The present invention also relates to a pharmaceutical composition comprising as active substance at least one compound of formula (I) as defined above, or a pharmaceutically acceptable salt, hydrate or solvate thereof, and at least one other inhibitor of the mTOR pathway selected from the group consisting of wortmannin, rapamycin, temsirolimus and everolimus, optionally in association with a pharmaceutically acceptable carrier or excipient.

The present invention also relates to products containing:
- at least one compound of formula (I) as defined above, or a pharmaceutically acceptable salt, hydrate or solvate thereof, and
- at least one other inhibitor of the mTOR pathway as defined above,
as a combined preparation for simultaneous, separate or sequential use in the prevention or treatment of a disease associated with a dysregulation of the mTOR pathway, wherein the disease associated with a dysregulation of the mTOR pathway is a hamartoma syndrome.

### Description of the invention

As intended herein, the term "comprising" has the meaning of "including" or "containing", which means that when an object "comprises" one or several elements, other elements than those mentioned may also be included in the object. In contrast, when an object is said to "consist of" one or several elements, the object is limited to the listed elements and cannot include other elements than those mentioned.

### Compound

Preferably, the above-defined compound of formula (I) is represented by the following formula (II): 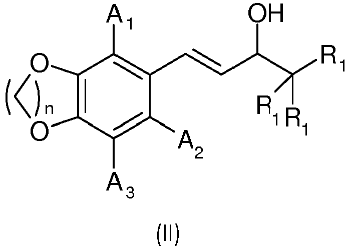 in which n, A₁, A₂, A₃ and R₁ are as defined above.

More preferably the above-defined compound of formula (I) or (II) is represented by the following formula (III), i.e. stiripentol:

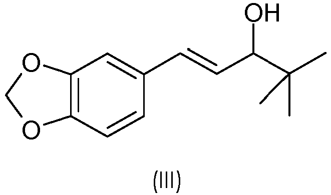

Preferred alkyl groups according to the invention encompass the methyl, ethyl, n-propyl, isopropyl, n-butyl, s-butyl and t-butyl groups.

The CI, I, Br or F atoms are preferred halogen atoms according to the invention.

French patent FR 2 173 691, which is incorporated herein by reference, describes the synthesis of stiripentol, in particular starting from methylenedioxy-3,4-phenyl-1-dimethyl-4,4-penten-1-on-3. It is well within the ordinary skills of one of skill in the art to synthesize the other compounds of formula (I) from this teaching.

As will be clear to one of skill in the art, the above-defined formulas (I), (II), and (III) represent either the various stereoisomers encompassed by these formulas or mixtures thereof, in particular racemic mixtures thereof.

Thus, the compound of formula (III) can be a compound of formula (Illa) a compound of formula (IIIb), or a mixture of a compound of formula (Illa) and a compound of formula (IIIb), in particular the racemic mixture thereof.

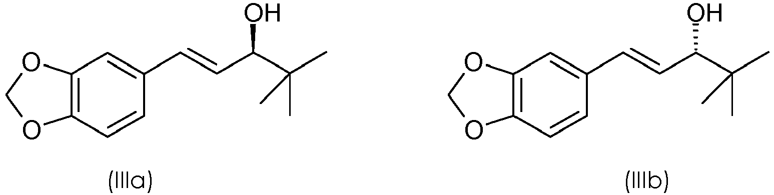

### Prevention and treatment

As intended herein the "mTOR pathway" relates to an intracellular signaling pathway regulating the cell cycle and involving the mTOR protein. "mTOR" relates to the mechanistic or mammalian Target Of Rapamycin and is also known as the FK506-binding protein 12-rapamycin-associated protein 1 (FRAP1). mTOR is encoded by the *MTOR* gene. As is well known to one of skill in the art, mTOR links with other proteins and serves as a core component of two distinct protein complexes, mTOR complex 1 (mTORC1) and mTOR complex 2 (mTORC2), which regulate different cellular processes. In particular, as a core component of both complexes, mTOR functions as a serine/threonine protein kinase that regulates cell growth, cell proliferation, cell motility, cell survival, protein synthesis, autophagy, and transcription. As a core component of mTORC2, mTOR also functions as a tyrosine protein kinase that promotes the activation of insulin receptors and insulin-like growth factor 1 receptors. mTORC2 has also been implicated in the control and maintenance of the actin cytoskeleton. Examples of upstream regulators of the mTOR pathway, i.e. regulators upstream of mTOR, notably encompass PI3K-AKT. Examples of downstream effectors of the mTOR pathway, i.e. effectors downstream of mTOR, notably encompass the S6 kinase which phosphorylates ribosomal protein S6.

As intended herein the expression "disease associated with a dysregulation of the mTOR pathway" relates to any disease in which the regulation of the mTOR pathway is impaired or absent. Preferably, the dysregulation of the mTOR pathway according to the invention relates to an activation, in particular a constitutive activation or a hyper activation of the mTOR pathway, or to a lack of inhibition, in particular a constitutive lack of inhibition, of the mTOR pathway. Preferably also the disease associated with a dysregulation of the mTOR pathway is a disease due to or caused by a dysregulation of the mTOR pathway.

The disease associated with a dysregulation of the mTOR pathway according to the invention is a hamartoma syndrome. More preferably, the disease associated with a dysregulation of the mTOR pathway according to the invention is selected from the group consisting of tuberous sclerosis, PTEN-related hamartoma syndrome and Peutz-Jeghers syndrome.

"Tuberous sclerosis" (TS) is well known to one of skill in the art. It is also known as "Bourneville tuberous sclerosis" (BTS), "Bourneville's disease" or "tuberous sclerosis complex" (TSC).

By way of example tuberous sclerosis is classified in paragraph Q85.1 of 10^{th} revision of the international classification of disease of the World Health Organization (ICD-10).

Besides, the diagnosis of tuberous sclerosis, which can be classified as definite, probable or possible diagnostic, can be effected as follows:
- definite diagnostic: two major criteria, or one major criteria and 2 minor criteria.
- probable diagnostic: one major criteria and one minor criteria.
- possible diagnostic: one major criteria, or at least two minor criteria.

### Major criteria:

- Angiofibroma of the face or forehead plate
- non-traumatic ungual fibromas or periungual fibromas
- hypomelanotic macules (more than 3)
- shagreen patches or multiple collagenomas
- multiples nodular retinal hamartomas
- cortical tuber^{(a)}
- subependymal nodules
- subependymal giant cell astrocytomas
- cardiac rhabdomyoma, single or multiple
- lymphangioleiomyomatosis or renal angiomyolipomas^{(b)}

### Minor criteria

- Randomly distributed pits in dental enamel
- Hamartomatous rectal polyps^{(c)}
- Bone cysts^{(d)}
- White matter migration lines^{(a,d,e)}
- Gingival fibroids
- Non-renal hamartomas^{(c)}
- Achromic flecked retina
- "confetti" skin lesions
- Multiple renal cysts

^{(a)}The coexistence of a cerebral cortical dysplasia and White matter migration lines counts as a criterion.
^{(b)}The coexistence of a lymphangioleiomyomatosis and a renal angiomyolipoma counts as a criterion.
^{(c)}A histological confirmation is suggested.
^{(d)}The radiological diagnostic is sufficient.

Tuberous sclerosis is an autosomal dominant disease. As such, tuberous sclerosis according to the invention is preferably associated to a mutation in the TSC1 gene and/or in the TSC2 gene.

As intended herein "PTEN" relates to Phosphatase and TENsin homolog. As is known in the art, the "PTEN-related hamartoma syndrome" is an autosomal dominant disease resulting from a mutation of the tumor suppressor gene PTEN. The PTEN-related hamartoma syndrome according to the invention in particular includes Cowden syndrome (CS), Bannayan-Riley-Ruvalcaba syndrome (BRRS), PTEN-related proteus syndrome (PS), and proteus-like syndrome.

The Peutz-Jeghers syndrome is well known in the art and results from a mutation in the STK11 tumor suppressor gene.

### Individual

The individual according to the invention is preferably a mammal, more preferably a human. Preferably also, the individual according to the invention is a child or an infant.

The individual according to the invention, preferably has one or more symptoms of the disease associated with a dysregulation of the mTOR pathway.

Preferably, the individual according to the invention presents a dysregulation of the mTOR pathway.

Preferably, the individual according to the invention presents at least one mutation in a gene selected from TSC1, TSC2, PTEN and SK11 genes.

Preferably also, the individual according to the invention preferably presents at least one symptom of tuberous sclerosis. Most preferably, the individual according to the invention is diagnosed with a definite tuberous sclerosis, or a probable tuberous sclerosis, or a possible tuberous sclerosis.

Preferably also, the individual according to the invention presents at least one symptom of a PTEN-related hamartoma syndrome. More preferably, the individual according to the invention has at least one symptoms selected from the group consisting of Cowden syndrome (CS) and/or at least one symptom of Bannayan-Riley-Ruvalcaba syndrome (BRRS) and/or at least one symptom of PTEN-related proteus syndrome (PS) and/or at least one symptom of proteus-like syndrome.

### Administration

Preferably, the compound of formula (I) as defined above or the pharmaceutically acceptable salt, hydrate or solvate thereof, is to be administered at a unit dose of from 100 mg to 1000 mg or from 5 mg/kg to 100 mg/kg. Preferably also, the compound of formula (I) as defined above or the pharmaceutically acceptable salt, hydrate or solvate thereof is to be administered with a dosage regimen of from 10 mg/kg/d to 200 mg/kg/d.

Preferably, the compound of formula (I) as defined above or the pharmaceutically acceptable salt, hydrate or solvate thereof, the pharmaceutical composition for use as defined above, the pharmaceutical composition as defined above or the medicament as defined above, is in a form suitable for administration by the oral route. Preferably also, the compound of formula (I) as defined above or the pharmaceutically acceptable salt, hydrate or solvate thereof, the pharmaceutical composition for use as defined above, the pharmaceutical composition as defined above or the medicament as defined above, is in the form of a powder, sachets, tablets or capsules.

As intended herein, "pharmaceutically acceptable carrier or excipient" refers to any material suitable with a pharmaceutical composition. Preferably, the pharmaceutically acceptable carrier or excipient according to the invention is suitable for an oral administration. Preferably, the pharmaceutically acceptable carrier or excipient according to the invention includes but is not limited to any of the standard of pharmaceutical composition known to one of skill in the art such as water, standard of pharmaceutical composition known to one of skill in the art such as water, glycerin, alcohol, oil emulsion, water emulsion, buffered saline solution, preservative, stabilizer and wetting agents.

### Additional compound

As intended herein, the expression "other inhibitor of the mTOR pathway" relates to any compound intended to alleviate one or more of the symptoms or to treat or prevent a disease associated to a dysregulation of the mTOR pathway. Preferably, the other inhibitor of the mTOR pathway is selected from the group consisting of wortmannin, rapamycin and the analogs of rapamycin, such as temsirolimus and everolimus.

As intended herein "combined" or "in combination" means that the compound of formula (I) as defined above or the pharmaceutically acceptable salt, hydrate or solvate thereof, in particular stiripentol, are administered at the same time than the additional compound, either together, *i.e*. at the same administration site, or separately, or at different times, provided that the time period during which the compound of formula (I) as defined above or the pharmaceutically acceptable salt, hydrate or solvate thereof exerts its pharmacological effects on the individual and the time period during which the additional compound exerts its pharmacological effects on the individual, at least partially intersect.

The description will be further described by the following non-limiting Figures and Example.

### Description of the figures

**Figure 1****:**

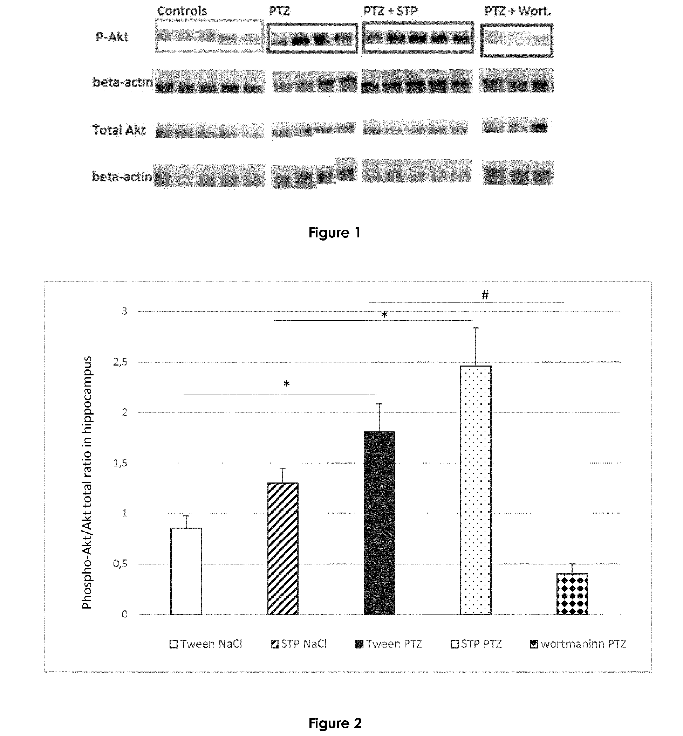

   Figure 1 represents the western blot analysis of the content in phosphorylated Akt (P-Akt) and total Akt of hippocampus from control rats treated with tween and saline (controls), rats treated with pentylenetetrazol and pretreated with vehicle (PTZ), rats treated with pentylenetetrazol and pretreated with stiripentol (PTZ + STP) and rats treated with pentylenetetrazol and pretreated with wortmannin (PTZ + Wort). Beta-actin is used as control.
**Figure 2****:**
   Figure 2 represents the ratio of phosphorylated Akt to total Akt (vertical axis) in the hippocampus of adult rats treated with NaCl pretreated with tween (n=5, white bar), NaCl pretreated with stiripentol (n=5, hatched bar), pentylenetetrazol pretreated with tween (n=4, black bar), pentylenetetrazol pretreated with stiripentol (n=5, doted bar) and pentylenetetrazol pretreated with wortmannin (n=3, diamond bar). Data are presented as the mean ± standard error of the mean.
   The star symbol (*) represents p<0,05 for pentylenetetrazol versus NaCl within either tween or stiripentol (two-way ANOVA followed by a Holm-Sidak multiple comparisons). The hash symbol (#) represents p<0,05 for pentylenetetrazol-wortmannin compared to pentylenetetrazol-tween (Student t-test).
**Figure 3****:**

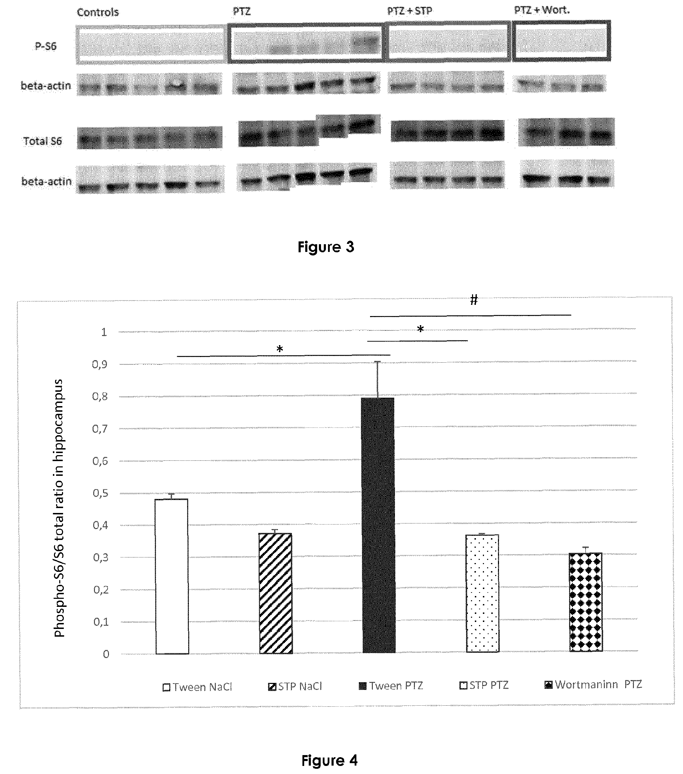

   Figure 3 represents the western blot analysis of the content in phosphorylated S6 (P-S6) and total S6 of hippocampus from control rats treated with tween and saline (controls), rats treated with pentylenetetrazol (PTZ), rats treated with pentylenetetrazol and pretreated with stiripentol (PTZ + STP) and rats treated with pentylenetetrazol and pretreated with wortmannin (PTZ + Wort.) Beta-actin is used as control.
**Figure 4****:**
   Figure 4 represents the ratio of phosphorylated S6 to total S6 (vertical axis) in the hippocampus of adult rats treated with NaCl pretreated with Tween (n=5, white bar), NaCl pretreated with stiripentol (n=5, hatched bar), pentylenetetrazol pretreated with tween (n=5, black bar), pentylenetetrazol pretreated with stiripentol (n=4, doted bar) and pentylenetetrazol pretreated wortmannin (n=3, diamond bar).

Data are presented as the mean ± standard error of the mean.

The star symbol (*) represents p<0.05 for pentylenetetrazol versus NaCl within the tween group and stiripentol versus vehicle within pentylenetetrazol group (two-way ANOVA followed by a Holm-Sidak multiple comparisons).

The hash symbol (#) represents p<0,05 for pentylenetetrazol-wortmannin compared to pentylenetetrazol-tween (Student t-test).

### EXAMPLE

The modulation of the mTOR pathway by the compound according to the invention, in particular stiripentol, was studied and compared with wortmannin, a pharmacological inhibitor of the mTOR pathway.

The mTOR pathway has been studied at two levels (i) an upstream level of regulation with the PI3K-Akt, a modulator of the mTOR pathway and (ii) a downstream level with the ribosomal protein S6, the substrate of the S6 kinase, a direct downstream effector of the mTOR pathway.

### A. Materials and methods

### 1. Animals

Adult male Sprague-Dawley rats (Janvier, 220-250 g, n=24, 7 weeks old) were used in this study. They were housed 2 per cage (Techniplast ref. 1291), and maintained in a 12 h light/dark cycle (light ON at 7 AM). Food and water were provided ad libitum. The experiments were conducted in accordance with the European Recommendations (directive 2010/63/EU) for the use and care of laboratory animals. The experimental protocol has been approved by the ethics committee.

### 2. Pharmacological treatment

Pentylenetetrazol (batch MKBV0751V, SIGMA), 80 mg/kg in 0.9% NaCl was administered subcutaneously in an injection volume of 5 mL/kg.

### 3. Test compounds

Wortmannin (Sigma), 2.4 mg/kg in DMSO 10% v/v in 0.9% NaCl or Stiripentol (Biocodex), 300 mg/kg in tween80 5% v/v, were injected intraperitoneally (10 mL/kg) 30 min before pentylenetetrazol administration.

### 4. Western blot

Thirty minutes after pentylenetetrazol administration, animals were sacrificed, the two hemi-hippocampi were isolated, homogenized in RIPA buffer (Abcam, ab 156034) containing a phosphatase and protease inhibitors cocktails (Abcam, ab201119) and frozen in liquid nitrogen.

The protein concentration of each sample was determined by a BCA protein assay. Equal amounts of total protein extract (20 µg) were separated by gel electrophoresis (Biorad 10% precast gel, ref 5671035, 14 µL deposit) and transferred to nitrocellulose membranes (Biorad ref. 1620167).

Membranes were incubated overnight at 4°C with primary antibodies to S6 Ribosomal Protein (5G10) (Rabbit mAb, 1:1000, Cell Signaling Technology, Danvers, MA), or Phospho-S6 Ribosomal Protein (D68F8) (Ser240/244) (Rabbit mAb, 1:1,000, Cell Signaling Technology, Danvers, MA), Akt (pan) (C67E7) (Rabbit mAb, 1:1000, Cell Signaling Technology, Danvers, MA), Phospho-Akt (Ser473) (D9E) (XP Rabbit mAb, Cell Signaling,).

Secondary Anti-Rabbit IgG, HRP-linked Antibody was then used.

Signals were detected by enzyme chemiluminescence (Vilber Lourmat Fusion FX5 system) and quantitatively analyzed with the Bio-1D software (Vilber Lourmat, France). The signal of phosphoprotein levels was normalized to the total protein.

Each membrane was also stripped (RestoreTM western blot stripping buffer, Thermoscientific) and reprobed to detect beta-actin protein levels, a control for the total quantity of protein deposited.

### 5. Data Analysis

Data are represented as the mean ± standard error of the mean (S.E.M.). The difference between groups was assessed by a two-way ANOVA followed by a Holm-Sidak multiple comparison (factor pretreatment= tween or STP, factor treatment= NaCl or PTZ).

The effect of wortmaninn was assessed by a t-test comparison between tween-pentylenetetrazol (n=5) versus wortmannin- pentylenetetrazol groups (n=3).

For all tests, significance was set at p<0.05 (Sigma Stat, v3.5, SPSS, Chicago, USA).

### B. Results

### 1. Effect of an acute stiripentol administration on Akt protein phosphorylation levels in the hippocampus

Western blots analysis revealed a strong band of phosphorylated Akt in either the pentylenetetrazol pretreated with vehicle groups or pentylenetetrazol pretreated with stiripentol group (**Figure 1****).** On the contrary, no band was visible in the pentylenetetrazol pretreated with wortmannin group. The levels of total Akt did not show any statistical difference between groups and provided a reference point to normalize all the data (median value=30 for all groups).

A pretreatment with stiripentol did not modify the pentylenetetrazol-induced increase in the ratio of phosphorylated Akt related to total Akt (no statistical difference between stiripentol- pentylenetetrazol (2.46) and tween- pentylenetetrazol (1.81), two-way ANOVA followed by a Holm-Sidak multiple comparisons), whereas the wortmannin treatment statistically significantly decrease the ratio of phosphorylated Akt related to total Akt in the pentylenetetrazol groups (p<0.05, Wortmannin-pentylenetetrazol (0.40) versus Vehicle-pentylenetetrazol (1.81) treated animals, Student t-test, **Figure 2****).**

### 2. Effect on an acute stiripentol administration on S6 protein phosphorylation levels in the hippocampus

Western blots analysis revealed a band of phosphorylated S6 protein in the PTZ group **(****Figure 3****).** No band was visible in either the PTZ pretreated with wortmannin group or the PTZ pretreated with stiripentol group. The levels of total S6 did not show any difference between groups and provided a reference point to normalize all the data (mean value from 0.9 to 1.3).

A pretreatment with STP statistically significantly blocked the increase in the ratio of phosphorylated S6 related to total S6 protein (p<0.05, STP (0.36) versus Veh-treated (0.79) animals within the PTZ group, two-way ANOVA followed by a Holm-Sidak multiple comparisons).

Similarly, a pretreatment with wortmannin blocked S6 phosphorylation level increase in the PTZ groups (p<0.05, Wortmannin-PTZ (0.30) versus Veh-PTZ (0.79) treated animals, Student t-test, **Figure 4****).**

These results suggest that stiripentol could inhibit the mTOR pathway at a specific level, downstream from the Akt level.

## Claims

1. A compound of the following formula (I): 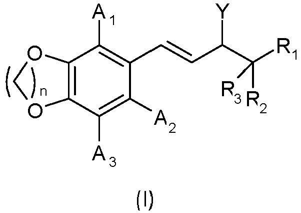 wherein:
- n represents 1 or 2,
- A₁, A₂ and A₃, which may be identical or different, represent a hydrogen atom, a halogen atom or a linear or branched alkyl group having from 1 to 4 carbon atoms,
- R₁, R₂ and R₃ represent independently a hydrogen atom or a linear or branched alkyl group having from 1 to 4 carbon atoms, and
- Y represents -OH, =O or -SH;
or a pharmaceutically acceptable salt, hydrate or solvate thereof,
for use in the prevention or treatment of a disease associated with a dysregulation of the mTOR pathway in an individual, wherein the disease associated with a dysregulation of the mTOR pathway is a hamartoma syndrome.

2. The compound or pharmaceutically acceptable salt, hydrate or solvate thereof for use according to claim 1, wherein the compound of formula (I) is of the following formula (II): 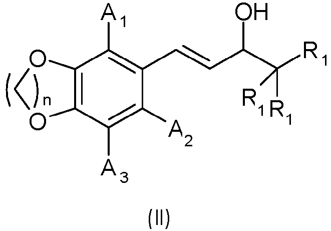 wherein n, A1, A2, A3 and R1 are as defined in claim 1.

3. The compound or pharmaceutically acceptable salt, hydrate or solvate thereof for use according to claim 1 or 2, wherein the compound of formula (I) is of the following formula (III):

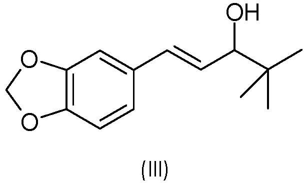

4. The compound or pharmaceutically acceptable salt, hydrate or solvate thereof for use according to any of claims 1 to 3, wherein the disease associated with a dysregulation of the mTOR pathway is selected from the group consisting of tuberous sclerosis, PTEN-related hamartoma syndrome and Peutz-Jeghers syndrome.

5. The compound or pharmaceutically acceptable salt, hydrate or solvate thereof for use according to any of claims 1 to 4, in combination with at least one other inhibitor of the mTOR pathway.

6. The compound or pharmaceutically acceptable salt, hydrate or solvate thereof for use according to any of claims 1 to 5, in combination with a least one other inhibitor of the mTOR pathway selected from the group consisting of wortmannin, rapamycin, temsirolimus and everolimus.

7. A pharmaceutical composition comprising:
- at least one compound of formula (I) as defined in any of claims 1 to 3, or a pharmaceutically acceptable salt, hydrate or solvate thereof, as active ingredient, and
- optionally at least one pharmaceutically acceptable carrier or excipient,
for use in the prevention or treatment of a disease associated with a dysregulation of the mTOR pathway as defined in any of claims 1 and 4 in an individual.

8. A pharmaceutical composition comprising:
- at least one compound of formula (I) as defined in any of claims 1 to 3, or a pharmaceutically acceptable salt, hydrate or solvate thereof, as active ingredient, and
- at least one other inhibitor of the mTOR pathway, as an active ingredient, and
- optionally at least one pharmaceutically acceptable carrier or excipient
wherein the at least one other inhibitor of the mTOR pathway is selected from the group consisting of wortmannin, rapamycin, temsirolimus and everolimus.

9. The pharmaceutical composition according to claim 8, for use in the prevention or treatment of a disease associated with a dysregulation of the mTOR pathway as defined in any of claims 1 and 4 in an individual.

10. Products containing:
- at least one compound of formula (I) as defined in any of claims 1 to 3, or a pharmaceutically acceptable salt, hydrate or solvate thereof, and
- at least one other inhibitor of the mTOR pathway,
as a combined preparation for simultaneous, separate or sequential use in the prevention or treatment of a disorder associated with a dysregulation of the mTOR pathway, as defined in any of claims 1 and 4 in an individual.

11. Products for use according to claim 10, wherein the at least one other inhibitor of the mTOR pathway is selected from the group consisting of wortmannin, rapamycin, temsirolimus and everolimus.

12. The compound or pharmaceutically acceptable salt, hydrate or solvate thereof, the pharmaceutical composition, or the products, for use according to any of claims 1 to 7 or 9 to 11, wherein the compound or pharmaceutically acceptable salt, hydrate or solvate thereof inhibits the mTOR pathway.

13. The compound or pharmaceutically acceptable salt, hydrate or solvate thereof, the pharmaceutical composition, or the products, for use according to any of claims 1 to 7 or 9 to 12, wherein the individual presents with at least one mutation in the TSC1 gene and/or in the TSC2 gene.

## Patentansprüche

1. Verbindung der folgenden Formel (I): 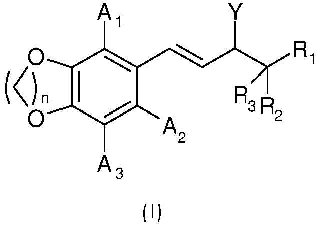 in der:
- n für 1 oder 2 steht,
- A₁, A₂ und A₃, die gleich oder verschieden sein können, für ein Wasserstoffatom, ein Halogenatom oder eine unverzweigte oder verzweigte Alkylgruppe mit 1 bis 4 Kohlenstoffatomen stehen,
- R₁, R₂ und R₃ unabhängig voneinander für ein Halogenatom oder eine unverzweigte oder verzweigte Alkylgruppe mit 1 bis 4 Kohlenstoffatomen stehen, und
- Y für -OH, =O oder -SH steht,
oder ein pharmazeutisch verträgliches Salz, Hydrat oder Solvat davon,
zum Gebrauch in der Vorbeugung oder Behandlung einer Krankheit, die mit einer Deregulierung des mTOR-Signalweges bei einem Patienten verbunden ist, wobei die mit einer Deregulierung des mTOR-Signalweges verbundene Krankheit ein Hamarton-Syndrom ist.

2. Verbindung oder pharmazeutisch verträgliches Salz, Hydrat oder Solvat davon zum Gebrauch nach Patentanspruch 1, wobei die Verbindung der Formel (I) der folgenden Formel (II) genügt: 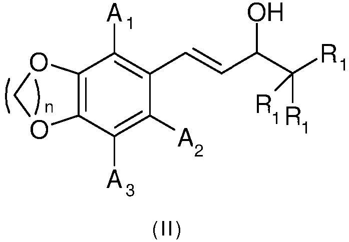 in der n, A₁, A₂, A₃ und R₁ wie in Patentanspruch 1 definiert sind.

3. Verbindung oder pharmazeutisch verträgliches Salz, Hydrat oder Solvat davon zum Gebrauch nach Patentanspruch 1 oder 2, wobei die Verbindung der Formel (I) der folgenden Formel (III) genügt:

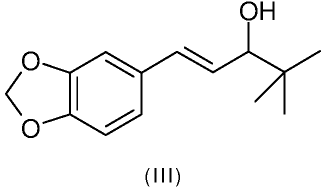

4. Verbindung oder pharmazeutisch verträgliches Salz, Hydrat oder Solvat davon zum Gebrauch nach irgendeinem der Patentansprüche 1 bis 3, wobei die mit einer Deregulierung des mTOR-Signalweges verbundene Krankheit aus der Gruppe, bestehend aus tuberöser Sklerose, PTEN-Hamarton-Tumor-Syndrom und Peutz-Jeghers-Syndrom gewählt wird.

5. Verbindung oder pharmazeutisch verträgliches Salz, Hydrat oder Solvat davon zum Gebrauch nach irgendeinem der Patentansprüche 1 bis 4 in Kombination mit mindestens einem anderen Inhibitor des mTOR-Signalweges.

6. Verbindung oder pharmazeutisch verträgliches Salz, Hydrat oder Solvat davon zum Gebrauch nach irgendeinem der Patentansprüche 1 bis 5, in Kombination mit mindestens einem anderen Inhibitor des mTOR-Signalweges, der aus der Gruppe, bestehend aus Wortmannin, Rapamycin, Temsirolimus und Everolimus gewählt wird.

7. Pharmazeutische Zubereitung, enthaltend:
- mindestens eine Verbindung der Formel (I) nach irgendeinem der Patentansprüche 1 bis 3, oder ein pharmazeutisch verträgliches Salz, Hydrat oder Solvat davon, als Wirkstoff, und
- optional mindestens einen pharmazeutisch verträglichen Träger oder Hilfsstoff,
zum Gebrauch in der Vorbeugung oder Behandlung einer Krankheit, die mit einer Deregulierung des mTOR-Signalweges verbunden ist, nach irgendeinem der Patentansprüche 1 bis 4, bei einem Patienten.

8. Pharmazeutische Zubereitung, enthaltend:
- mindestens eine Verbindung der Formel (I) nach irgendeinem der Patentansprüche 1 bis 3, oder ein pharmazeutisch verträgliches Salz, Hydrat oder Solvat davon, als Wirkstoff, und
- mindestens einen anderen Inhibitor des mTOR-Signalweges als Wirkstoff, und
- optional mindestens einen pharmazeutisch verträglichen Träger oder Hilfsstoff,
wobei der mindestens eine andere Inhibitor des mTOR-Signalweges aus der Gruppe, bestehend aus Wortmannin, Rapamycin, Temsirolimus und Everolimus, gewählt wird.

9. Pharmazeutische Zubereitung nach Patentanspruch 8 zum Gebrauch in der Vorbeugung oder Behandlung einer Krankheit, die mit einer Deregulierung des mTOR-Signalweges verbunden ist, nach irgendeinem der Patentansprüche 1 bis 4, bei einem Patienten.

10. Produkte, umfassend:
- mindestens eine Verbindung der Formel (I) nach irgendeinem der Patentansprüche 1 bis 3, oder ein pharmazeutisch verträgliches Salz, Hydrat oder Solvat davon, als Wirkstoff, und
- mindestens einen anderen Inhibitor des mTOR-Signalweges,
als Kombinationspräparat zum gleichzeitigen, getrennten oder aufeinanderfolgenden Gebrauch in der Vorbeugung oder Behandlung einer Krankheit, die mit einer Deregulierung des mTOR-Signalweges verbunden ist, nach irgendeinem der Patentansprüche 1 bis 4, bei einem Patienten.

11. Produkte zum Gebrauch nach Patentanspruch 10, in denen der mindestens eine andere Inhibitor des mTOR-Signalweges aus der Gruppe, bestehend aus Wortmannin, Rapamycin, Temsirolimus und Everolimus gewählt wird.

12. Verbindung oder pharmazeutisch verträgliches Salz, Hydrat oder Solvat davon, pharmazeutische Zubereitung oder Produkte zum Gebrauch nach irgendeinem der Patentansprüche 1 bis 7 oder 9 bis 11, in denen die Verbindung oder das pharmazeutisch verträgliche Salz, Hydrat oder Solvat davon den mTOR-Signalweg inhibiert.

13. Verbindung oder pharmazeutisch verträgliches Salz, Hydrat oder Solvat davon, pharmazeutische Zubereitung oder Produkte zum Gebrauch nach irgendeinem der Patentansprüche 1 bis 7 oder 9 bis 12, wobei der Patient mindestens eine Mutation im TSC1-Gen und/oder im TSC2-Gen aufweist.

## Revendications

1. Composé de formule suivante (I) : 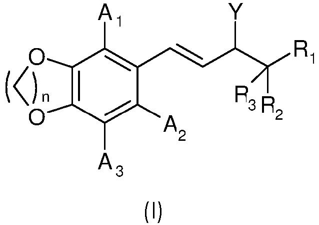 dans lequel :
- n représente 1 ou 2,
- A1, A2 et A3, qui peuvent être identiques ou différents, représentent un atome d'hydrogène, un atome d'halogène ou un groupe alkyle linéaire ou ramifié ayant 1 à 4 atomes de carbone,
- R1, R2 et R3 représentent indépendamment un atome d'hydrogène ou un groupe alkyle linéaire ou ramifié ayant 1 à 4 atomes de carbone, et
- Y représente -OH, =O ou -SH ;
ou un sel, hydrate ou solvate pharmaceutiquement acceptable de celui-ci,
pour son utilisation dans la prévention ou le traitement d'une maladie associée à une dérégulation de la voie mTOR chez un individu, dans laquelle la maladie associée à une dérégulation de la voie mTOR est un syndrome d'hamartome.

2. Composé ou sel, hydrate ou solvate pharmaceutiquement acceptable de celui-ci pour son utilisation selon la revendication 1, dans laquelle le composé de formule (I) est de formule suivante (II) : 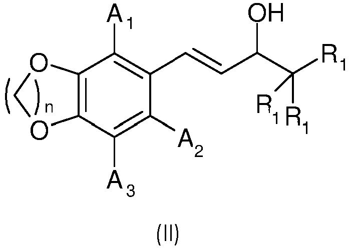 dans laquelle n, A1, A2, A3 et R1 sont tels que définis dans la revendication 1.

3. Composé ou sel, hydrate ou solvate pharmaceutiquement acceptable de celui-ci pour son utilisation selon la revendication 1 ou 2, dans laquelle le composé de formule (I) est de formule suivante (III) :

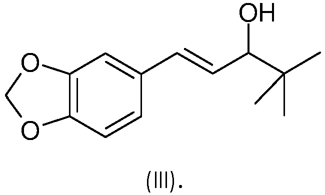

4. Composé ou sel, hydrate ou solvate pharmaceutiquement acceptable de celui-ci pour son utilisation selon l'une des revendications 1 à 3, dans laquelle la maladie associée à une dérégulation de la voie mTOR est sélectionnée dans le groupe constitué de la sclérose tubéreuse, du syndrome d'hamartome lié à PTEN et du syndrome de Peutz-Jeghers.

5. Composé ou sel, hydrate ou solvate pharmaceutiquement acceptable de celui-ci pour son utilisation selon l'une des revendications 1 à 4, en combinaison avec au moins un autre inhibiteur de la voie mTOR.

6. Composé ou sel, hydrate ou solvate pharmaceutiquement acceptable de celui-ci pour son utilisation selon l'une des revendications 1 à 5, en combinaison avec au moins un autre inhibiteur de la voie mTOR sélectionné dans le groupe constitué de la wortmannine, de la rapamycine, du temsirolimus et de l'évérolimus.

7. Composition pharmaceutique comprenant :
- au moins un composé de formule (I) tel que défini dans l'une des revendications 1 à 3, ou un sel, hydrate ou solvate pharmaceutiquement acceptable de celui-ci, en tant qu'ingrédient actif, et
- éventuellement au moins un véhicule ou un excipient pharmaceutiquement acceptable,
pour une utilisation dans la prévention ou le traitement d'une maladie associée à une dérégulation de la voie mTOR telle que définie dans l'une des revendications 1 et 4 chez un individu.

8. Composition pharmaceutique comprenant :
- au moins un composé de formule (I) tel que défini dans l'une des revendications 1 à 3, ou un sel, hydrate ou solvate pharmaceutiquement acceptable de celui-ci, en tant qu'ingrédient actif, et
- au moins un autre inhibiteur de la voie mTOR, en tant qu'ingrédient actif, et
- éventuellement, au moins un véhicule ou excipient pharmaceutiquement acceptable
dans laquelle l'au moins un autre inhibiteur de la voie mTOR est sélectionné dans le groupe constitué de la wortmannine, la rapamycine, le temsirolimus et l'évérolimus.

9. Composition pharmaceutique selon la revendication 8, pour son utilisation dans la prévention ou le traitement d'une maladie associée à une dérégulation de la voie mTOR telle que définie dans l'une des revendications 1 et 4 chez un individu.

10. Produits contenant :
- au moins un composé de formule (I) tel que défini dans l'une des revendications 1 à 3, ou un sel, hydrate ou solvate pharmaceutiquement acceptable de celui-ci, et
- au moins un autre inhibiteur de la voie mTOR,
en tant que préparation combinée pour une utilisation simultanée, séparée ou séquentielle dans la prévention ou le traitement d'un trouble associé à une dérégulation de la voie mTOR, telle que définie dans l'une des revendications 1 et 4 chez un individu.

11. Produits pour une utilisation selon la revendication 10, dans lesquels l'au moins un autre inhibiteur de la voie mTOR est sélectionné dans le groupe constitué par la wortmannine, la rapamycine, le temsirolimus et l'évérolimus.

12. Composé ou sel, hydrate ou solvate pharmaceutiquement acceptable de celui-ci, composition pharmaceutique ou produits, pour une utilisation selon l'une des revendications 1 à 7 ou 9 à 11, dans laquelle le composé ou le sel, hydrate ou solvate pharmaceutiquement acceptable de celui-ci inhibe la voie mTOR.

13. Composé ou sel, hydrate ou solvate pharmaceutiquement acceptable de celui-ci, composition pharmaceutique ou produits, pour une utilisation selon l'une des revendications 1 à 7 ou 9 à 12, dans laquelle l'individu présente au moins une mutation dans le gène TSC1 et/ou dans le gène TSC2.
